# EUROPEAN PATENT SPECIFICATION

(11) **EP 0 443 688 B1**
(45) Date of publication and mention of the grant of the patent: **06.05.1998**
(21) Application number: 91200370.4
(22) Date of filing: 20.02.1991
(51) Int. Cl.: C07D 519/00, C08K 5/3415

(54) **Spirodilactam-arylsulfone oligomers**
Spirodilactam-Arylsulfon-Oligomere
Oligomères de spirodilactame-arylsulfone

(30) Priority: 20.02.1990 US 482099
(43) Date of publication of application: 28.08.1991
(73) Proprietor: SHELL INTERNATIONALE RESEARCH MAATSCHAPPIJ B.V., 2596 HR Den Haag (NL)
(72) Inventor: Wang, Pen-Chung, Houston, Texas 77079 (US); Chuah, Hoe Hin, Houston, Texas 77095 (US)

(56) References cited:
- EP-A- 359 341
- EP-A- 0 336 475
- EP-A- 0 384 518
- US-A- 4 562 243
- US-A- 4 847 388
- US-A- 4 968 810

## Description

This invention relates to a novel class of spirodilactam-arylsulfone oligomers having chain terminating groups comprising carbon to carbon or carbon to nitrogen unsaturation.

Unsaturated ether or ester derivatives of oligomers of polyhydric phenol ethers are well known as a class of compounds that can be cured or crosslinked to produce insoluble products which exhibit good solvent resistance and mechanical properties as well as high heat distortion temperatures. Such unsaturated ethers and esters are crosslinked to produce tough, heat resistant products for example in U.S. patent 4,701,514, which are processed by conventional methods into sheets, laminates with fiber glass or other reinforcements, or shaped articles and the crosslinked products are also useful in adhesive formulations.

The novel products of the invention are oligomers of the formula

E―O-[―(SL)-O―R―SO₂―R―O]ₙ―(SL)―O―E (I)

wherein n is a number of from 3 to 8, inclusive; E is an alkenyl, vinylarylmethyl or benzocyclobutenealkyl group having up to 10 carbon atoms and terminal carbon to carbon unsaturation; R is aryl with one or two aromatic rings and up to 15 carbon atoms; and SL is a moiety of the formula 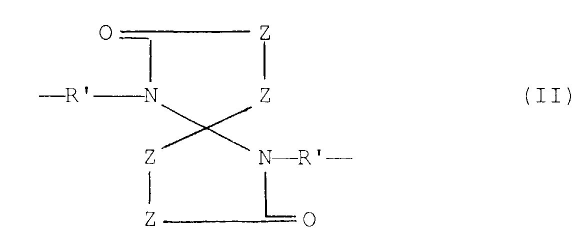 wherein each Z independently represents >C(Z')₂ in which each Z' independently represents hydrogen or lower alkyl; and R' has the same meanings as R.

In the present novel oligomers the spirodilactam moieties of formula II can be derived from compounds such as 1,6-di(4-hydroxyphenyl)-1,6-diazaspiro[4.4]-nonane-2,7-dione, 1,6-di(3-hydroxy-4-chlorophenyl)-3,8-dimethyl-1,6-diazapiro[4.4]nonane-2,7-dione, 1,6-di[4-(4-hydroxybenzyl)-phenyl]-1,6-diazaspiro[4.4]-nonane-2,7-dione, 1,6-di(4-hydroxyphenyl)-3,3,4,4,8,8,9,9-octamethyl-1,6-diazaspiro[4.4]nonane-2,7-dione, 1,6-di [4-(4'-hydroxybiphenyl)]-3,3-dimethyl-1, 6-diazaspiro[4.4]-nonane-2,7-dione, 1,6-di[2-(4-hydroxyphenyl)propyl]1, 6-diazaspiro-[4.4]nonane-2,7-dione, 1,6-di[4-(4-hydroxyphenylisopropyl)phenyl]-1,6-diazaspiro[4.4]nonane-2,7-dione.

The spirodilactam moieties of the above formula II and their methods of preparation are known from EP-A 336475 and EP-A 359341.

Starting from one of the spirodilactam moieties set out herein the oligomers of this invention are obtained in a two-step process, the first step of which is concerned with producing oligomers comprising arylsulfone and spirodilactam moieties only and then, in the second step, reacting the latter oligomers with appropriate compounds to introduce the defined terminating groups into the oligomer molecules. In the first step a dihydroxyterminated spirodilactam compound is reacted with a di(halo-aryl)sulfone in the presence of an alkaline compound to remove hydrohalogen acid, e.g. HCl or HBr.

Suitable di(halo-aryl)sulfone reactants used in the first reaction step are those comprising fluoro, chloro, bromo or iodo atoms, but preferably comprise chlorine or bromine. The halogen substituents on each aromatic ring are preferably ortho or para, or mixtures thereof. Illustrative of the halo-aryl sulfone reactants are di(4-chlorophenyl) sulfone, 4-chlorophenyl 4-bromophenyl sulfone, 1-(4-chloro-naphthyl) 4-fluorophenyl sulfone, and di(4-chloro-3-methylphenyl) sulfone.

Suitable alkaline compounds are alkali metal bases, e.g., the hydroxide, carbonate or bicarbonate of lithium, potassium or sodium. These are reacted with the hydroxy terminate spirodilactam compounds to produce the alkali metal salt thereof whilst removing water from the reaction mixture, e.g. by conventional methods such as extraction or distillation. A preferred method of water removal is by azeotropic distillation with a portion of the reaction diluent. Dimethyl sulfoxide is a preferred diluent for this purpose although other diluents such as N-alkylamides, e.g., N,N-dimethylformamide, N,N-dimethylacetamide, N-methyl-2-pyrrolidone or the like, also in mixtures with alkylated benzenes, such as toluene or ethylbenzene, are also satisfactory. Subsequent to the reaction with the alkali metal base, the alkali metal salt(s) may be recovered by conventional methods such as solvent removal or precipitation.

The halo-aryl sulfone reactant is present in a molar ratio of from 3:1 to 1:3 with the total molar quantity of alkali metal salt reactants. However, the sulfone reactant is preferably present in a substantially stoichiometric quantity, i.e., a molar ratio of sulfone reactant to total alkali metal salt reactant of substantially 1:1.

Oligomerization takes place at an elevated temperature, typically from 80°C to 225°C but more often from 100°C to 220°C. The pressure is sufficient to maintain the reaction mixture in a liquid phase. Such pressures are up to 2MPa. (20 atmospheres), preferably up to lMPa. (10 atmospheres). During oligomerization, the reactant contact is maintained by conventional methods such as stirring or shaking and subsequent to reaction the oligomer product is recovered by well-known techniques, such as solvent removal, precipitation or extraction and the like.

The process to produce the spirodilactam-terminated polyether sulfone oligomer is conducted under oligomerization conditions in the liquid phase in an inert reaction diluent. Preferred diluents are polar diluents capable of dissolving at least a portion of each reactant at oligomerization temperature. In a particularly useful procedure, the oligomerization diluent is the same as the diluent employed in alkali metal salt production, but such is not required.

The resulting oligomers have an average formula weight of from 1,000 to 40,000, preferably from 5,000 to 30,000 and, more preferably from 10,000 to 20,000.

The unsaturated derivatives produced in the last reaction step are ether derivatives of the hydroxyaryl substituents in the terminal spirodilactam units of the oligomer. The unsaturated moiety, which becomes bound to the oxygen of the oxyaryl moiety (derived by loss of hydrogen from the terminal hydroxyaryl moiety of the spirodilactam end units of the oligomer), is an alkenyl, vinylarylmethyl or benzocyclobutenealkyl group having up 10 carbon atoms and terminal carbon to carbon unsaturation. Although unsaturated moieties of conventional types are useful in the ether derivatives of the invention, the preferred unsaturated ether moieties are selected from 2-alkenyl, such as allyl, methallyl, and crotyl; vinylarylmethyl, such as 4-styrylmethyl and 4-vinyl-2-methylbenzyl; and benzocyclobutenealkyl. Preferred unsaturated moieties are allyl, 4-vinylbenzyl and benzocyclobutenemethyl.

These derivatives are tvpically produced by reacting a compound containing the desired unsaturated moiety with an alkali metal salt of the hydroxyaryl-substituted spirodilactam-terminated polyether sulfone oligomer. Although lithium, sodium, potassium, rubidium and cesium salts of the hydroxyaryl-substituted spirodilactam-terminated polyether sulfone oligomers are usefully employed in the production of the unsaturated ether derivatives of the invention, the use of a sodium salt or a potassium salt is preferred. In one embodiment, the alkali metal salt of the hydroxyaryl-substituted spirodilactam-terminated polyether sulfone oligomer is produced by contacting the spirodilactam-terminated polyether sulfone oligomer with a substantially stoichiometric quantity of alkali metal hydroxide, i.e., substantially 2 moles of alkali metal hydroxide for each mole of the spirodilactam-terminated polyether sulfone oligomer. Sodium or potassium hydroxide is preferred. Reaction is conducted in the liquid phase in a suitable reaction solvent such as N-methyl-2-pyrrolidinone, N,N-dimethylacetamide, N,N-dimethylformamide or the like while removing the water present or formed by distillation, preferably azeotropic distillation employing a second solvent, such as toluene or ethylbenzene, with which water forms an azeotrope. The use of an alkali metal hydroxide is not specifically required and employment of an equivalent amount of alkali metal carbonate or bicarbonate is satisfactory. The alkali metal salt of the hydroxyaryl-substituted spirodilactam-terminated polyether sulfone oligomer is isolated if desired by conventional procedures, such as solvent removal, but the salt is typically used in situ in the media of its production for reaction with the compound containing the unsaturated moiety.

Thus, the second step of the process of this invention can be defined as a process for producing an oligomer as defined in this invention which comprises reacting in the liquid phase (a) an unsaturated compound of the formula E-G wherein E is as defined above and G is halo or alkoxy, and (b) a hydroxyaryl-substituted [4.4] spirodilactam-terminated polyether sulfone oligomer having nitrogen atoms in the 1- and 6-positions of the spiro ring system and having a hydroxyaryl substituent on each terminal spiro ring nitrogen atom.

G is halo, preferably chlorine or bromine, or alkoxy, preferably alkoxy of up to 4 carbon atoms. When an ether derivative of the hydroxyaryl-substituted spirodilactam-terminated polyether sulfone oligomer is desired the E moiety is preferably allyl, 4-styrylmethyl, or benzocyclobutenemethyl and is generally provided as the halide. Allyl chloride, allyl bromide, p-vinylbenzyl chloride, or chloromethylbenzocyclobutene are illustrative.

The reaction of the alkali metal salt of the hydroxyaryl-substituted spirodilactam-terminated polyether sulfone oligomer and the E-G compound is conducted in liquid phase solution in the presence of a reaction diluent. Preferred diluents are polar diluents in which the compounds undergoing are soluble, at least at reaction conditions. Suitable reaction diluents include N-alkylamides such as N,N-dimethylacetamide, N,N-dimethylformamide and N-methyl-2-pyrrolidone, phenols such as phenol and m-cresol and sulfur containing diluents such as sulfolane and dimethylsulfoxide or the like. The compound which provides the unsaturated moiety, i.e., the compound E-G, is utilized in a molar amount equal to or in excess over the alkali metal salt. Molar ratios from 5:1 to 1:1 are suitable. The stoichiometry of the reaction would suggest reaction of the E-G compound and the alkali metal salt in a 2:1 ratio. Molar ratios of from 3:1 to 1.5:1 are preferred.

Reaction is effected by charging the unsaturated moiety compound, the alkali metal salt of the hydroxyaryl-substituted spirodilactam-terminated polyether sulfone oligomer and the reaction diluent to a suitable reactor and maintaining the reaction mixture under reaction conditions. Alternatively, the alkali metal salt is employed as produced in the media of its production and the E-G compound is added to the solution of the alkali metal salt if produced in a suitable reaction diluent.

Reaction to produce the unsaturated ether derivatives of the hydroxyaryl-substituted spirodilactam-terminated polyether sulfone oligomer is conducted over a range of reaction conditions, typically including a reaction temperature of from -30°C to 200°C, preferably from -10°C to 175°C, the higher portion of the temperature range being preferred. A suitable reaction pressure is one which will maintain the reaction mixture in the liquid phase. Such pressures are typically up to 2MPa. (20 atmospheres) but more often are from 80 to 500 kPa (0.8 atmosphere to 5 atmospheres).

The ether derivatives of the hydroxy-substituted spirodilactams-terminated polyether sulfone oligomers find utility as thermosetting resins which are employed in the production of the cured or crosslinked products of the invention. These products are useful as surface coatings, in adhesive formulations and in fiber-reinforced composites wherein, for example, the fiber is glass or carbon. The cured products are also useful in the production of hollow objects as by filament winding and are employed as impregnating and casting resins. The processing of the cured products for these applications is by conventional methods.

The curing of the unsaturated ether derivatives is accomplished by conventional methods such as thermal curing, e.g., heating to a temperature above 200°C, by photochemical excitation, e.g., as by exposure to high energy radiation, by catalyzed polymerization employing cationic or anionic catalysts or by reaction with a polyfunctional curing agent. Anionic polymerization uses alkali metal alkoxides, hydroxides or amides as the catalyst typical cationic polymerization catalysts are organic or inorganic acids or are Lewis acids. Such cationic catalysts include sulfuric acid, phosphonic acid, p-toluenesulfonic acid, boron trifluoride, tin tetrachloride and the like. Catalysts are generally employed in a quantity of from 0.05 to 5% by weight, based on total composition. In an alternate modification, the unsaturated ether or ester derivatives are cured by heating with a substantial amount, e.g., from 20 to 50% by weight, based on total composition, of a polyfunctional curing agent.

In the present invention the preferred cured products are obtained by reacting the unsaturated ether derivatives with a polyfunctional curing agent. Such curing agents are organic compounds having at least two substituents with multiple bonds between adjacent atoms. Such substituents are hydrocarbyl with multiple bonds between adjacent carbon atoms or are non-hydrocarbyl with multiple bonds between atoms at least one of which is not a carbon atom. Preferred polyfunctional curing agents have up to 30 carbon atoms inclusive and contain functional groups selected from alkenyl, alkynyl, styrylmethyl, cyanato or maleimido. Particularly preferred are the maleimido-substituted polyfunctional curing agents, especially di(4-maleimidophenyl)methane. The other classes of polyfunctional curing agents are also well known in the art, and include such preferred curing agents as triallyl isocyanurate, di(4-cyanatophenyl)methane, 2,2-di(4-cyanatophenyl)propane and the like.

The invention is further illustrated by the following Illustrative Embodiments which should not be construed as limiting the invention.

### Example 1

To a three liter three-necked flask was added a mixture of 63.14 g (0.22 mole) of di-4-chlorophenyl sulfone, 81.12 g (0.24 mole) of 1,6-di(4-hydroxyphenyl)-1,6-diazaspiro[4.4]nonane-2,7-dione, 34.55 g (0.25 mole) of potassium carbonate, 100 ml of toluene and 200 ml N-methyl-2-pyrrolidinone (NMP). The mixture was heated to 150°-160°C and water removed by azeotropic distillation. When the water removal was complete, the temperature was lowered to 80°-90°C and 7.0 g of chloromethylbenzocyclobutene in 50 ml of NMP was added at 70-80°C over the next 6 hours. The resulting mixture was cooled and filtered. The filtrates were poured slowly into water. The precipitated benzocyclobutene-terminated oligomer product was recovered by filtration and dried in a vacuum oven at 80°C.

Moulding of the oligomer product cured at 295°C for 3 minutes gave a cured material having a glass transition temperature of 287°C.

### Example 2

The product of Illustrative Embodiment I was mixed 17 parts by weight with 83 parts by weight of bismaleimide, i.e., di(4-maleimidophenyl)methane. The resulting mixture was melted at 175°C and then heated to 200°C for 3 hours and finally at 250°C for 5 hours. The resulting cured product was insoluble in common solvents and had a glass transition temperature in excess 350°C.

### Example 3

To a three liter three-necked flask was added a mixture of 63.14 g (0.22 mole) of di-4-chlorophenyl sulfone, 81.12 g (0.2 mole) of 1,6-di(4-hydroxyphenyl)-1,6-diazaspiro[4.4]nonane-2,7-dione, 34.55 g (0.25 mole) of potassium carbonate, 200 ml of N-methyl-2-pyrrolidinone (NMP) and 100 ml of toluene. The mixture was heated to 150°-160°C and the water was removed by azeotropic distillation. When the water removal was complete, the temperature was lowered to 80°-90°C and 5.0 g of allyl bromide in 50 ml of NMP was added at 70°-80°C over a 6-hour period. The resulting solution was then cooled, filtered and the filtrates were poured slowly into water to give a precipitated allyl-terminated oligomer product which was recovered by filtration and dried in a vacuum oven at 80°C.

Mouldings of this allyl-terminated oligomer product, cured by heating for 3 minutes at 290°C, gave a cured product which had a glass transition temperature of 239°C.

### Example 4

A mixture of 17 parts by weight of the oligomer of example 3 and 83 parts by weight of di(4-maleimidophenyl)methane was melted at 190°-200°C and then heated in an oven in a first stage to 200°C for 3 hours and in a second stage at 265°C for an additional 5 hours. The resulting cured product had a glass transition temperature in excess of 350°C.

### Example 5

Following procedures similar to those described in examples 1 and 3 above, 7.0 g of chloromethylstyrene was used in place of chloromethylbenzocyclobutene or allyl bromide and the desired oligomer product recovered.

Moulding of this styrene-terminated oligomer cured at 290°C for 3 minutes gave a cured material having a glass transition temperature of 260°C.

## Claims (Claims for the following Contracting State(s): BE, CH, DE, FR, GB, IT, LI, NL)

1. An oligomer of the formula
E―O-[―(SL)-O―R―SO₂―R―O]ₙ―(SL)―O―E (I)
wherein n is a number of from 3 to 8, inclusive; E is an alkenyl, vinylarylmethyl or benzocyclobutenealkyl group having up to 10 carbon atoms and terminal carbon to carbon unsaturation; R is aryl with one or two aromatic rings and up to 15 carbon atoms; and SL is a moiety of the formula 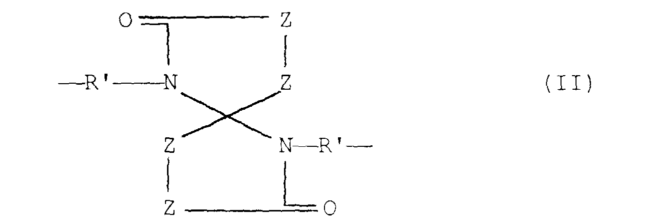 wherein each Z independently represents >C(Z')₂ in which each Z' independently represents hydrogen or lower alkyl; and R' has the same meanings as R.

2. The oligomer according to claim 1 wherein each E independently represents allyl, 4-styrylmethyl, or benzocyclobutenemethyl.

3. The derivative of claim 1 or 2 wherein each Z is >C(H)₂.

4. The process of producing an oligomer as claimed in any one of claims 1 to 3 which comprises reacting in the liquid phase (a) an unsaturated compound of the formula E-G wherein E is as defined in claim 1 and G is halo or alkoxy, and (b) a hydroxyaryl-substituted [4.4] spirodilactam-terminated polyether sulfone oligomer having nitrogen atoms in the 1- and 6- positions of the spiro ring system and having a hydroxyaryl substituent on each terminal spiro ring nitrogen atom.

5. A thermosetting resin composition comprising an oligomer as claimed in any one of claims 1 to 3 and a maleimido curing agent.

## Claims (Claims for the following Contracting State(s): ES)

1. The process of producing an oligomer of the formula
E-O⁅(SL)-O―R―SO₂―R―O]ₙ―(SL)―O―E (I)
wherein n is a number of from 3 to 8, inclusive; E is an alkenyl, vinylarylmethyl or benzocyclobutenealkyl group having up to 10 carbon atoms and terminal carbon to carbon unsaturation; R is aryl with one or two aromatic rings and up to 15 carbon atoms; and SL is a moiety of the formula 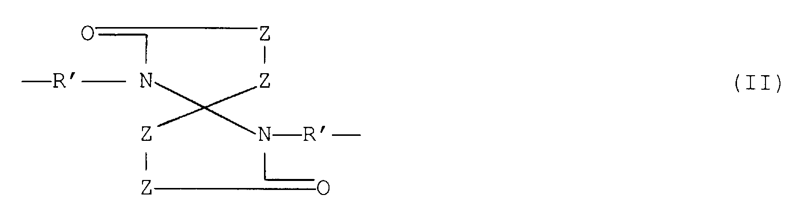 wherein each Z independently represents >C(Z')₂ in which each Z' independently represents hydrogen or lower alkyl; and R' has the same meanings as R, which comprises reacting in the liquid phase (a) an unsaturated compound of the formula E-G wherein E is as defined above and G is halo or alkoxy, and (b) a hydroxyaryl-substituted [4.4] spirodilactam-terminated polyether sulfone oligomer having nitrogen atoms in the 1- and 6- positions of the spiro ring system and having a hydroxyaryl substituent on each terminal spiro ring nitrogen atom.

2. The process of producing the oligomer according to claim 1 wherein each E independently represents allyl, 4-styrylmethyl, or benzocyclobutenemethyl.

3. The process of producing the derivative of claim 1 or 2 wherein each Z is >C(H)₂.

4. The process of producing a thermosetting resin composition by combining an oligomer as produced by a process as claimed in any one of claims 1 to 3 and a maleimido curing agent.

## Patentansprüche (Patentansprüche für folgende(n) Vertragsstaat(en): BE, CH, LI, DE, FR, GB, IT, NL)

1. Ein Oligomer mit der Formel
E-O-[-(SL)-O-R-SO₂-R-O]ₙ-(SL)-O-E (I),
worin n eine Zahl von 3 bis einschließlich 8 bedeutet, E eine Alkenyl-, vinylarylmethyl- oder Benzocyclobutenalkylgruppe mit bis zu 10 Kohlenstoffatomen und einer endständigen Kohlenstoff-Kohlenstoff-Unsättigung bedeutet; R für Aryl mit einem oder mit zwei aromatischen Ringen und bis zu 15 Kohlenstoffatomen steht; und SL einen Rest der Formel 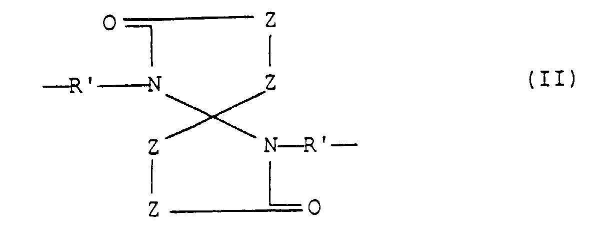 bedeutet, worin jedes Z unabhängig voneinander für >C(Z')₂ steht, worin jedes Z' unabhängig voneinander Wasserstoff oder Niederalkyl bedeutet; und R' die gleichen Bedeutungen wie R aufweist.

2. Oligomer nach Anspruch 1, worin jedes E unabhängig voneinander für Allyl, 4-Styrylmethyl oder Benzocyclobutenmethyl steht.

3. Derivat nach Anspruch 1 oder 2, worin jedes Z für >C(H)₂ steht.

4. Verfahren zur Herstellung eines Oligomers nach einem der Ansprüche 1 bis 3, welches ein in flüssiger Phase erfolgendes Umsetzen von (a) einer ungesättigten Verbindung der Formel E-G, worin E wie in Anspruch 1 definiert ist und G für Halogen oder Alkoxy steht, mit (b) einem Hydroxyarylsubstituierten, [4.4] Spirodilactam-terminierten Polyethersulfonoligomer mit Stickstoffatomen in den 1- und 6-Stellungen des Spiroringsystems und mit einem Hydroxyarylsubstituenten an jedem endständigen Spiroring-Stickstoffatom umfaßt.

5. Warmhärtende Harzzusammensetzung, umfassend ein Oligomer nach einem der Ansprüche 1 bis 3 und ein Maleimido-Härtungsmittel.

## Patentansprüche (Patentansprüche für folgende(n) Vertragsstaat(en): ES)

1. Verfahren zur Herstellung eines Oligomers mit der Formel
E-O-[-(SL)-O-R-SO₂-R-O]ₙ-(SL)-O-E (I),
worin n eine Zahl von 3 bis einschließlich 8 bedeutet, E eine Alkenyl-, Vinylarylmethyl- oder Benzocyclobutenalkylgruppe mit bis zu 10 Kohlenstoffatomen und einer endständigen Kohlenstoff-Kohlenstoff-Unsättigung steht; R für Aryl mit einem oder mit zwei aromatischen Ringen und bis zu 15 Kohlenstoffatomen steht; und SL einen Rest der Formel 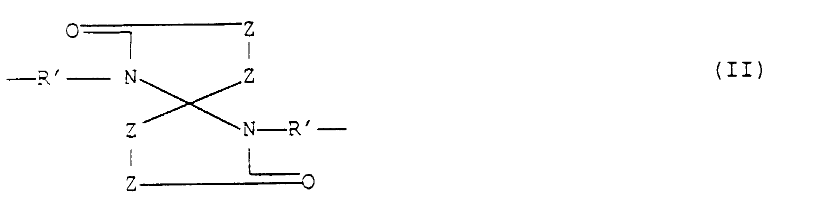 bedeutet, worin jedes Z unabhängig voneinander für >C(Z^{'})₂ steht, worin jedes Z' unabhängig voneinander Wasserstoff oder Niederalkyl bedeutet; und R' die gleichen Bedeutungen wie R aufweist, welches ein in flüssiger Phase erfolgendes Umsetzen von (a) einer ungesättigten Verbindung der Formel E-G, worin E wie oben definiert ist und G für Halogen oder Alkoxy steht, mit (b) einem Hydroxyaryl-substituierten, [4.4] Spirodilactam-terminierten Polyethersulfonoligomer mit Stickstoffatomen in den 1- und 6-Stellungen des Spiroringsystems und mit einem Hydroxyarylsubstituenten an jedem endständigen Spiroring-Stickstoffatom umfaßt.

2. Verfahren zur Herstellung des Oligomers nach Anspruch 1, worin jedes E unabhängig voneinander für Allyl, 4-Styrylmethyl oder Benzocyclobutenmethyl steht.

3. Verfahren zur Herstellung des Derivats nach Anspruch 1 oder 2, worin jedes Z für >C(H)₂ steht.

4. Verfahren zur Herstellung einer warmhärtenden Harzzusammensetzung durch Vereinigen eines Oligomers, wie nach einem Verfahren der Ansprüche 1 bis 3 gebildet, mit einem Maleimido-Härtungsmittel.

## Revendications (Revendications pour l'(les) Etat(s) contractant(s) suivant(s): BE, CH, LI, DE, FR, GB, IT, NL)

1. Oligomère de la formule
E―O-[―(SL)-O―R―SO₂―R―O]ₙ―(SL)―O―E (I)
dans laquelle n représente un nombre dont la valeur varie de 3 à 8 inclusivement, E représente un radical alcényle, vinylarylméthyle ou benzocyclobutènealkyle, possédant jusqu'à 10 atomes de carbone et une insaturation carbone à carbone terminale, R représente un groupe aryle qui comporte un ou deux noyaux aromatiques et jusqu'à 15 atomes de carbone et SL représente un groupement de la formule 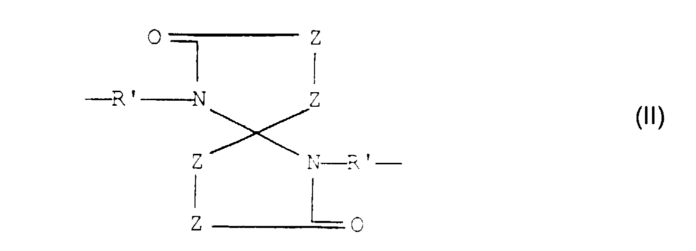 dans laquelle chaque symbole Z représente >C(Z')₂ où chaque symbole Z' représente indépendamment un atome d'hydrogène ou un radical alkyle inférieur et R' possède les mêmes significations que R.

2. Oligomère suivant la revendication 1, caractérisé en ce que chaque symbole E représente indépendamment un radical allyle, 4-styrylméthyle ou benzocyclobutèneméthyle.

3. Dérivé suivant la revendication 1 ou 2, caractérisé en ce que chaque symbole Z représente >C(H)₂.

4. Procédé de production d'un oligomère suivant l'une quelconque des revendications 1 à 3, caractérisé en ce que l'on fait réagir, en phase liquide, (a) un composé insaturé de la formule E-G dans laquelle E possède les significations qui lui ont été attribuées dans la revendication 1 et G représente un halogène ou un radical alcoxy et (b) un oligomère de polyéthersulfone à terminaison [4.4]spirodilactame hydroxyarylsubstituée dans les positions 1 et 6 du système cyclique du type spiro et possédant un substituant du type hydroxyaryle sur chaque atome d'azote du cycle spiro terminal.

5. Composition de résine thermodurcissable, qui comprend un oligomère suivant l'une quelconque des revendications 1 à 3 et un agent de durcissement du type maléimido.

## Revendications (Revendications pour l'(les) Etat(s) contractant(s) suivant(s): ES)

1. Procédé de production d'un oligomère de la formule
E―O-[―(SL)-O―R―SO₂―R―O]ₙ―(SL)―O―E (I)
dans laquelle n représente un nombre dont la valeur varie de 3 à 8 inclusivement, E représente un radical alcényle, vinylarylméthyle ou benzocyclobutènealkyle, possédant jusqu'à 10 atomes de carbone et une insaturation carbone à carbone terminale, R représente un groupe aryle qui comporte un ou deux noyaux aromatiques et jusqu'à 15 atomes de carbone et SL représente un groupement de la formule 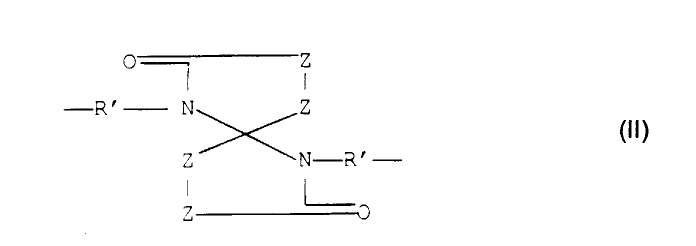 dans laquelle chaque symbole Z représente >C(Z')₂ où chaque symbole Z' représente indépendamment un atome d'hydrogène ou un radical alkyle inférieur et R' possède les mêmes significations que R, caractérisé en ce que l'on fait réagir, en phase liquide, (a) un composé insaturé de la formule E-G dans laquelle E possède les significations qui lui ont été attribuées ci-dessus et G représente un halogène ou un radical alcoxy et (b) un oligomère de polyéthersulfone à terminaison [4.4]spirodilactame hydroxyarylsubstituée dans les positions 1 et 6 du système cyclique du type spiro et possédant un substituant du type hydroxyaryle sur chaque atome d'azote du cycle spiro terminal.

2. Procédé de production de l'oligomère suivant la revendication 1, caractérisé en ce que chaque symbole E représente indépendamment un radical allyle, 4-styrylméthyle ou benzocyclobutèneméthyle.

3. Procédé de production d'un dérivé suivant la revendication 1 ou 2, caractérisé en ce que chaque symbole Z représente >C(H)₂.

4. Procédé de production d'une composition de résine thermodurcissable par la combinaison d'un oligomère tel que produit par un procédé suivant l'une quelconque des revendications 1 à 3 et d'un agent de durcissement du type maléimido.
